# EUROPÄISCHE PATENTANMELDUNG

(11) **EP 0 852 146 A2**
(43) Veröffentlichungstag der Anmeldung: **08.07.1998**
(21) Anmeldenummer: 98600001.6
(22) Anmeldetag: 05.01.1998
(51) Int. Cl.: A61L 2/06, A61L 2/26, A61L 11/00

(54) **Sterilisation - Desinfektion ohne sekundäre Infektion.**

(30) Priorität: 03.01.1997 GR 97100001
(71) Anmelder: Markatis, Spiridon, 11471 Athens (GR)
(72) Erfinder: Markatis, Spiridon, 11471 Athens (GR)

(57) **Zusammenfassung**

Anordnung fuer den Schutz der Umwelt vor sekundaerer Infektion, die von Ausgangsstoffen der Autoklaven fluessiger Sterilisation (mit Dampf) stammt, mit dem Zusatz eines Verdampfers, eines Vorfilters und eines Sterifilters fuer Gase, zwischen dem Abfluss des Autoklaven und der Vakuum-Einheit (KondensatorVakuumpumpe) eingeschaltet ist, sodass auf der einen Seite die fluessigen Abfaelle verdampfen, auf der anderen Seite die Gase (Luft und Dampf) durch Filtrieren sterilisiert werden, und danach in die Umwelt gelangen.

Sterilisationsprozess mit einem Autoklaven fluessiger Sterilisation, der einen Verdampfer und spezielle Filter beinhaltet, sowie einen geeigneten Sterilisationsprozess fuer den Verdampfer und den Filter.

Moeglichkeit der Wasserrueckgewinnung fuer die Funktion des Dampfgenerators in der Anordnung eines Autoklaven fluessiger Sterilisation, der ueber die empfohlene Erfindung fuer den Umweltschutz vor sekundaerer Infektion verfuegt.

Art und Weise der Entsorgung von infektioesen Abfaellen, der die Umwelt vor einer Infektion oder den Produkten aus deren Verbrennung schuetzt. Dies geschiet durch Sterilisation oder Desinfektion der infektioesen Abfaelle in mobilen oder stationaeren Einheiten, die ueber einen Autoklaven fluessiger Sterilisation mit der empfohlenen Erfindung ueber die Anordnung fuer den Umweltschutz vor sekundaerer Infektion verfuegen.

Behaelter, geeignet fuer die Beladung von Autoklaven fluessiger Sterilisation mit infektioesen Abfaellen zwecks Sterilisierung oder Desinfizierung, sodass Dampf eindringen, jedoch keine Fluessigkeiten oder Feststoffe von den Seitenwaenden ausdringen koennen, die Fluessigkeiten jedoch nur durch seine Grundflaeche hinausfliessen koennen und nur dann, wenn sich der Behaelter an seinem richtigen Platz in der Sterilisationskammer befindet.

## Beschreibung

Die Erfindung bezieht sich auf die Vorrichtung eines Autoklaven fluessiger Sterilisation-Desinfektion (mit Dampf), mit dem Zusatz eines Verdampfers, eines Vorfilters, eines Sterilisationsfilters und einer solchen Sterilisierungsprozedur, sodass waehrend des Sterilisationsprozesses und hauptsaechlich waehrend der Phase des Vorvakuums kein Schmutzwasser oder schmutzige Luft nach aussen in die Umwelt gelangt (Abwasserkanaele und Atmosphaere). Ebenfalls bezieht sie sich auf die Herstellung eines geeigneten Behaelters fuer Autoklaven, damit der infektioese Abfall der Krankenhaeuser sterilisiert oder desinfiziert werden kann, sowie auf ein Wasserruekgewinnungs - System des eingesetzten Wassers.

Die Autoklaven fluessiger Sterilisation-Desinfektion, die zur Sterilisation verschiedener Gegenstaende in den Krankenhaeusern oder in der Arzneiindustrie gebraucht werden, werden seit vielen Jahren angewandt. Um eine richtige Sterilisation mit Dampf durchzufuehren, muss die ganze atmosphaerische Luft vom Raum des Autoklaven, wie auch von der sterilisierten Ladung des Autoklaven ausgepresst werden.

Dies wird in der Phase des Vorvakuums erzielt, d.h. die Erzeugung von Vakuum niedriger als 0.2 bar absoluter Druck, Einlassung von Dampf bis zum absoluten Druck 1 oder 2 bar und Wiederholung des Prozesses 3 bis 4 mal.

Danach wird in den Raum Dampf eingelassen bis die noetige Temperatur fuer die Sterilisation erzielt wird (z.B. bei 134 Grad Celsius absoluter Druck des Dampfes 3.1 bar). Dies bildet die Phase der Sterilisation, dauert 5 - 10 Minuten, je nach der erwuenschten Durchfuehrung der Sterilisation, und waehrend dieser Phase wird die Temperatur stabil beibehalten.

In der naechsten Phase wird der Dampf wieder herausbefoerdert, es wird wieder fuer 5 - 10 Minuten ein Vakuum niedriger als 0.2 bar erzeugt, das als Trocken¶ Vakuum-Phase bezeichnet wird, und dies wird gemacht, damit die bereits sterilisierte Ladung trocknet.

Zuletzt folgt die Wiederherstellungsphase des atmosphaerischen Drucks im Raum des Autoklaven, das mit der Einlassung sterilisierter Luft durch ein antibakterielles Sterilfilter ermoeglicht wird (mit Loechern 0,2 µm).

Mit dem o.e. Verfahren funktionieren alle Autoklaven fluessiger Sterilisation, wobei jedoch die folgenden Probleme entstehen, die groesser sind, wenn die Gegenstaende, die sterilisiert werden sollen, bereits infiziert sind, was meistens der Fall ist.

1. In der Vakuum-Phase ist die Luft, die aus dem Raum und aus der zu sterilisierenden Ladung austritt, bereits infiziert, da sie mit der infektioesen Ladung in Kontakt gekommen ist. Bei den bestehenden Systemen wird diese infektioese Luft in die Atmosphaere befoerdert ohne weitere Behandlung.

2. Der Dampf, der waehrend der Vorvakuum-Phase eingelassen wird, wird kondensiert, weil die Ladung noch kalt ist, und wird verfluessigt. Bei den bestehenden Systemen werden diese Kondensate vom Autoklaven ohne weitere Behandlung hinausbefoerdert und durch die Vakuum-Einheit (Vakuumpumpe) gelangen sie in die Abwasserkanaele. Diese Kondensate sind mit der infektioesen Ladung in Kontakt gekommen, und zwar in fluessiger Umgebung, und sind somit infiziert.

3. Zuletzt wird mit den bestehenden Systemen der Autoklaven fluessiger Sterilisation sehr viel Wasser verschwendet, sowohl von den Kondensaten, als auch vom Wasser, das fuer den Betrieb der Vakuumpumpe benoetigt wird, die eine Fluessigkeitsring - Vakuumpumpe ist (geeignet fuer den Gebrauch in Dampf). Da das Wasser infiziert ist, weil es mit den infektioesen Kondensaten und der Luft in Kontakt gekommen ist, kann es nicht wieder verwendet werden und gelangt in die Abwasserkanaele. Dies ist fuer die Ortschaften ein grosses Problem, die Probleme mit der Wasserversorgung haben, aber auch fuer die, die zu hartes Wasser haben, die dann fuer den Betrieb des Autoklaven das Wasser enthaerten muessen.

4. Ein grosses Problem in den Krankenhaeusern ist die Behandlung des infektioesen Abfalls, der fuer die Gesundheit der Menschen im allgemeinen sehr gefaehrlich ist. Es ist nicht erlaubt, den Abfall dieser Art (OP's, Labors, stationaeren Kliniken usw.) durch bewohnte Ortschaften zu transportieren, sowie diesen Abfall mit dem Hausmuell ohne Behandlung zu vermischen, sodass der Transport sowie die Entsorgung schwierig ist.

Eine Art, die angewandt wird, ist die Verbrennung in einem Einaescherungs-Autoklaven mit beschraenkter Moeglichkeit an Verbrennungen pro Tag. Die Zusammensetzung dieses Abfalls ist zu 80 - 90% aus Kunststoff. Dies bedeutet, dass die Verbrennung dieses Abfalls aus Kunststoff, die bereits erhoehte Umweltbelastung noch mehr erschwert.

Eine Loesung dieses Problems waere die Sterilisation-Desinfektion des infektioesen Abfalls vor Ort, d.h. im Krankenhaus, mit einer stationaeren oder mobilen Einheit, die ueber einen gewoehnlichen Autoklaven fluessiger Desinfektion verfuegt, der nur fuer infektioese Abfaelle benutzt werden koennte. Danach koennte dieser Abfall zusammen mit dem haeuslichen Abfall abgefahren werden, da er jetzt ungefaehrlich ist. Die bereits bestehenden Systeme der Autoklaven fuer fluessige Desinfektion koennen nicht verwendet werden, aus den Gruenden, die in Par. 1 und 2 erwaehnt werden, und nicht nur deshalb. Der Raum des Autoklaven wuerde schnell mit verschiedenen krankenhaeuslichen Abfaellen gefuellt werden (Blut, Spritzen, Nadeln, Kunststoffusw.), die den Betrieb des Autoklaven erschweren und unaesthetisch gestalten wuerden.

Mit der betreffenden Erfindung werden alle o.e. Probleme geloest. D.h. auf der einen Seite werden die ausgestossenen Gase der Autoklaven fluessiger Desinfektion bevor sie in die Atmosphaere abgestossen werden, durch Filtrieren sterilisiert, zum andern werden die fluessigen Ausgangsstoffe in Gase umgewandelt, sterilisiert, filtriert, kondensiert und wieder eingesetzt, falls dies erwuenscht ist.

Mit dieser Erfindung kann man infektioese Abfaelle in Autoklaven fluessiger Sterilisation desinfizieren, die danach voellig desinfiziert sind, und mit dem normalen Hausmuell entsorgt werden koennen. Dabei ist keine besondere Reinigung des Raums der Sterilisation notwendig, wie diese in den Nachteilen oben erwaehnt wird.

Insbesondere wird gegenueber der Verbrennung der infektioesen Abfaelle in einem Einaescherungs-Autoklaven die Umwelt nicht mit Abgasen und Feststoffen (Staubpartikel, Asche) vom Typ Aerosol belastet, die zu einem grossen Teil zum photochemischen Smog beitragen.

Die Erfindung beruht auf den Zusatz eines Verdampfers, eines Vorfilters und eines Sterilfilters zwischen dem Abfluss der Sterilisationskammer des Autoklaven (fluessiger Sterilisation) und der Vakuum-Einheit (Kondensator, Vakuumpumpe), eingeschaltet ist, sodass der Abfall der Autoklaven fluessiger Sterilisation verdunstet und gefiltert (durch die speziellen Filter) wird und dann in die Vakuum-Einheit kommt und zuletzt in die Umwelt, befreit von Infektionen, gelangt.

Das System kann ebenso das bereits sterilisierte Wasser recyceln, das aus der Vakuum-Einheit des Autoklaven fluessiger Sterilisation ausfliesst, sodass es wieder im Dampfgenerator verwendet werden kann, wenn dies erwuenscht ist.

Die Erfindung beinhaltet ebenso einen Lade-Entladebehaelter fuer Autoklaven fluessiger Sterilisation fuer infektioese Abfaelle, so eingerichtet, dass die festen infektioesen Abfaelle, zum Schutz der Sterilisationkammer, nicht aus dem Ladebehaelter herausfallen koennen. Die Seitenwaende des Ladebehaelters sind abnehmbar, sodass er leichter gereinigt und be-und-entladen werden kann.

Die Erfindung beinhaltet ebenfalls einen geeigneten Sterilisationsprozess der Ladung des Autoklaven, indem der Verdampfer und die speziellen Filter zusammenarbeiten, sowie einen Sterilisationsprozess fuer den Verdampfer und die speziellen Filter.

Die Erfindung kann ebenfalls einen Behandlungsprozess fuer das recyceln des infektionsfreien Wassers beinhalten, sodass es dem Dampfgenerator zugefuehrt wird.

Figur 1 zeigt einen Block-Diagramm der Anwendung der Erfindung.

Figur 2 zeigt einen Verdampfer-Typ mit den speziellen Filtern (Vorfilter und Sterilfilter).

Figur 3 zeigt das hydraulische Diagramm eines bestehenden Autoklaven fluessiger Sterilisation.

Figur 4 zeigt das hydraulische Diagramm eines Autoklaven fluessiger Sterilisation mit dem Zusatz des Verdampfers, der speziellen Filter (Vorfilter und Sterilfilter), sowie eine Wasserrueckgewinnungsanlange.

Figur 5 zeigt die Druckdiagramme der Kammern des Autoklaven (Wellentyp A) und des Verdampfers (Wellentyp B), waehrend eines Sterilisations-Zyklus.

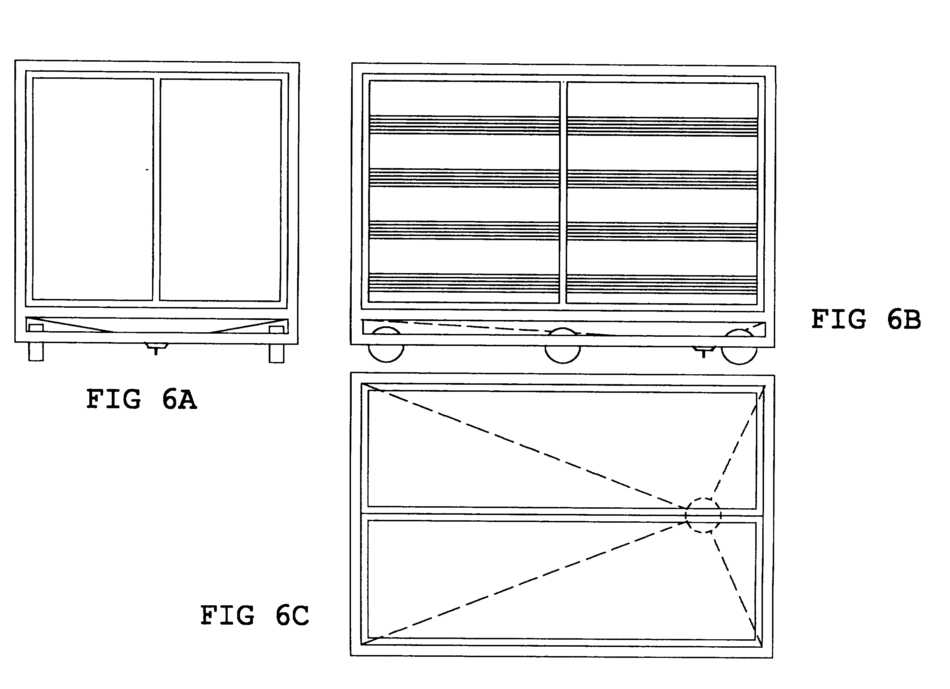

Figur 6A, 6B und 6C zeigen die Ansichten: Nebenseiten, Vorderseite und den Grundriss eines entsprechenden Ladebehaelters fuer infektioese Abfaelle in einem rechteckigen Autoklaven fluessiger Sterilisation.

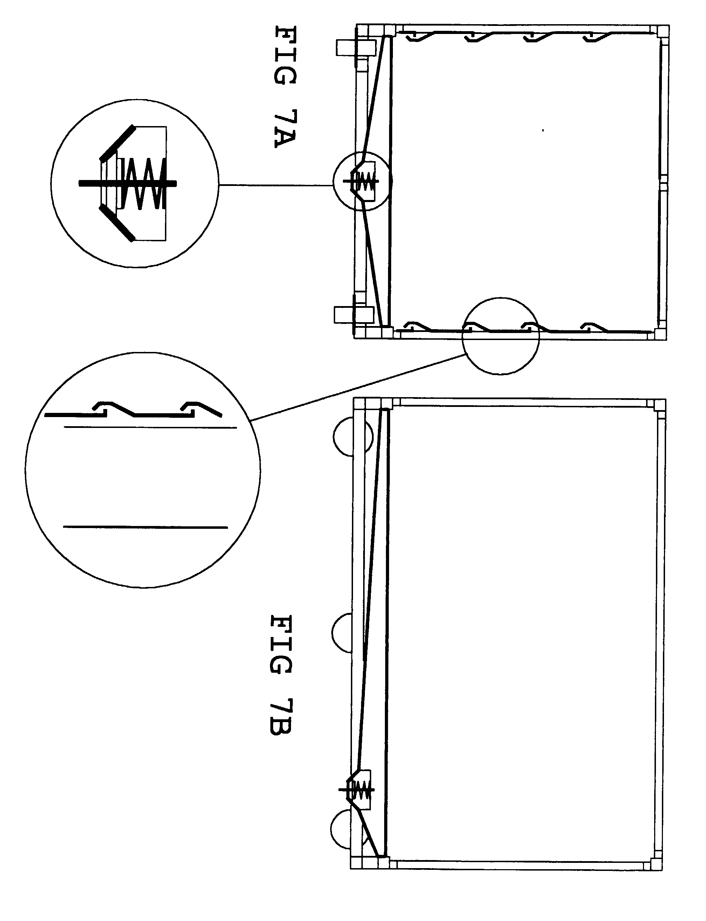

Figur 7A und 7B zeigen die Nebenseiten eines Ladebehaelters infektioesem Abfalls in einem rechteckigen Autoklaven fluessiger Sterilisation im Schnitt.

Figur 8 zeigt eine dreidimensionale Ansicht eines Ladebehaelters eines Autoklaven fluessiger Sterilisation, geeignet fuer die Sterilisation infektioesen Abfalls.

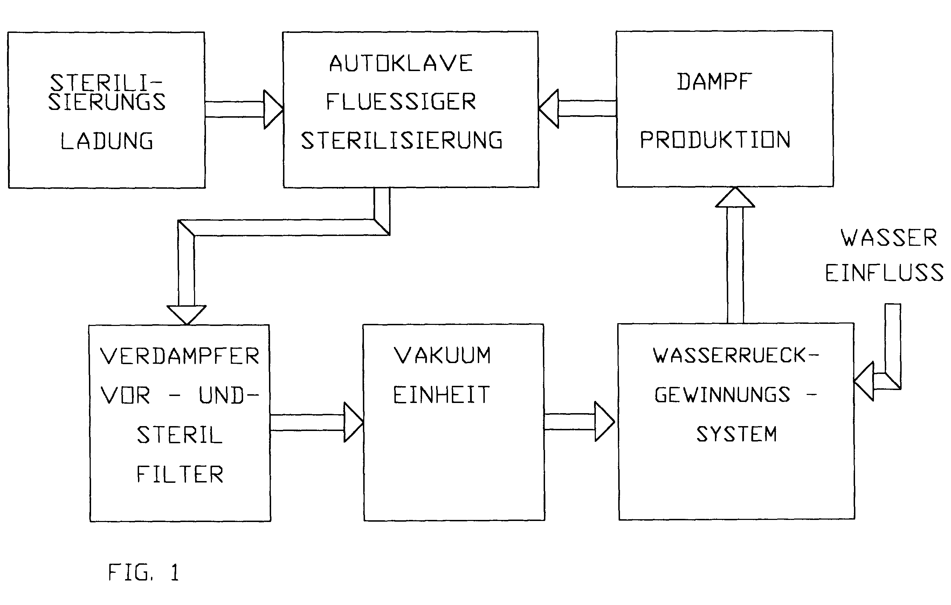

In Figur 1 sieht man einen Block-Diagramm eines Autoklaven-Systems fluessiger Sterilisation ohne sekundaere Infektion. Die zu sterilisierende Ladung wird in den Autoklaven eingeschoben. Wenn die zu sterilisierende Ladung infektioese Abfaelle sind, werden sie in den speziellen Ladebehaelter geladen, der in der Erfindung erwaehnt wird.

Um die Sterilisation durchzufuehren brauch man Dampf, der in den Autoklaven ueber den Dampfgenerator (Dampfproduktion) eingelassen wird. Die Ausgangsstoffe des Sterilisations-Autoklaven (Luft, Kondensate) kommen in den Verdampfer und in die speziellen Filter (Vorfilter, Sterilfilter), um abgedampft und filtriert zu werden. Der Verdampfer und der Filter ergibt den eigentlichen Teil der Erfindung.

Der Dampf und die Luft, die nun durch das Filtern sterilisiert worden sind, kommen in die Vakuum-Einheit (Kondensator, Vakuumpumpe). Das Wasser, das aus der Vakuum-Einheit ausgelassen wird, kann nun, da es nicht infiziert ist, wieder ueber die Wasserrueckgewinungsanlange fuer die Dampfproduktion (Dampfgenerator) verwendet werden. Bei den bestehenden Systemen der Autoklaven fluessiger Sterilisation werden die Ausgangsstoffe der Autoklaven direkt in die Vakuum-Einheit weitergeleitet und dann in die Atmosphaere und in die Abwasserkanaele, ohne vorherige Behandlung.

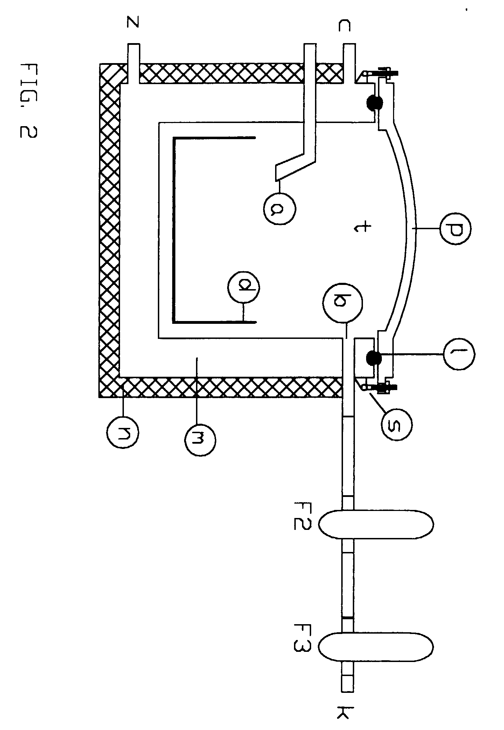

In Figur 2 wird ein Typ eines Verdampfers gezeigt mit den speziellen Filter (Vor-und Sterilfilter), damit seine Funktion verstaendlich wird. Der Verdampfer kann eine andere Form, als die, die beschrieben wird, oder eine andere Heizungsart haben.

Der Verdampfer kann ein Kessel aus rostfreiem Material sein, druckfest, temperaturfest und saeurefest. Er muss aussen mit einer Thermoisolierung waermeisoliert sein (**n**). Er verfuegt ueber eine Tuer (**p**), die luftdicht mit einem Gummi (**l**) schliesst, das temperaturfest ist und mit Schrauben (**s**) ringsherum schliesst.

Es ist eigentlich ein kleiner parallelflaechiger oder zylindrischer Autoklav, ca. 5% des Fassungsvermoegens des hauptsaechlichen Sterilisations-Autoklaven, ohne dass ein anderes Fassungsvermoegen ausgeschlossen wird. Der Verdampfer braucht eine Waermequelle, sodass die Fluessigkeit, die sich in seinem Boden befindet, verdampfen kann. Zu diesem Zweck kann es eine doppelte Wand (**m**) (Mantel) geben, wie auch beim hauptsaechlichen Autoklaven, an der sich staendig Dampfunter einem Druck von 2,5-3 bar (d.h. Temperatur 138-140 Grad C) befindet. Die Einfuehrung von Dampf zu diesem Zweck in den Mantel erfolgt an Punkt (**c**), zum anderen werden die Kondensate und der kalte Dampf an Punkt (**z**) ueber eine thermostatische Dampffalle herausgelassen, die an diesem Punkt eingebracht ist.

Die Waermequelle koennen ebenso elektrische Widerstandsdraehte am Boden des Verdampfers sein und nicht Dampf in der doppelten Wand oder eine sonstige Waermequelle, sodass die Temperatur des Verdampfers ungefaehr bestaendig bleibt.

Im Verdampfer befindet sich ein geeigneter Behaelter (**d**), in dem sich die Fluessigkeit sammelt, die vom Eingang des Verdampfers (**a**) in die Verdampferkammer (**t**) fliesst, sodass die festen Reste nach dem Verdampfen im Behaelter bleiben. Dieser Behaelter kann regelmaessig gereinigt werden, zumal diese Reste sterilisiert sind.

Der Eingang (**a**) der Kammer des Verdampfers ist mit dem Abfluss der Kammer des hauptsaechlichen Autoklaven durch ein Ventil verbunden. Somit gelangt alles, was von der Kammer des Sterilisations-Autoklaven (Luft, Fluessigkeit) herauskommt, in den Verdampfer.

Der Abfluss (**b**) der Kammer des Verdampfers befindet sich oben, sodass nur Gase (Luft, Dampf) herauskommen koennen.

Die Fluessigkeit, die vom Autoklaven in den Verdampfer fliesst, wird zu Dampf. Dies wird viel leichter und schneller erzielt, weil im Verdampfer immer ein Vakuum herrscht.

Der im Verdampfer erzeugte Dampf, wie auch der Dampf oder die Luft, die von der Kammer des Autoklaven kommen, geht durch das Vorfilter (**F2**), das eventuelle zurueckbleibende feste Stoffe festhaelt, damit ein Schaden des Sterilfilters, was nachgeschaltet ist, vermieden wird.

Danach gehen sie durch das Sterilfilter (**F3**), das ein Membranfilter ist und nur das Durchlaufen einer Masse kleiner als 0,2µm erlaubt, zumal der Filterdurchmesser 0.2µm ist. Filter dieser Art werden in Krankenhaeusern sowie in Produktionsstatten zur Produktion von Gasen, die zum aerztlichen Gebrauch vorgesehen sind, genutzt, um die Sterilisation der Gase zu erreichen.

Das Sterilfilter (Punkt **k**) ist durch ein Ventil mit der Vakuum-Einheit (Kondensat und Fluessigkeitsring - Vakuumpumpe) verbunden.

Mit dem geeigneten Prozess werden der Verdampfer und die speziellen Filter durch Dampf sterilisiert. Die Filter sind verbrauchbar und muessen, gemaess der Anweisung des Herstellers, nach einer bestimmten Anzahl an Sterilisationen ersetzt werden.

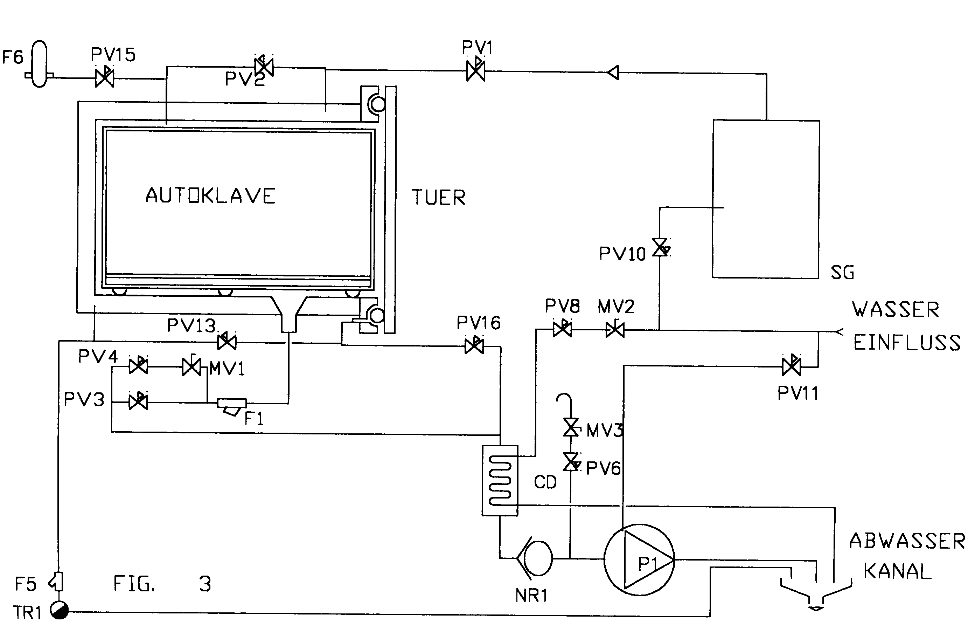

In Figur 3 sieht man die hydraulischer Kreislauf eines bestehenden Systems fluessiger Sterilisation, das hier zum Vergleich mit der empfohlenen Anordnung fluessiger Sterilisation gezeigt wird. Es ist zu ersehen, dass die Abfaelle des Sterilisations-Autoklaven in die Abwasserkanaele oder in die Atmosphaere gelangen, ohne weitere Behandlung.

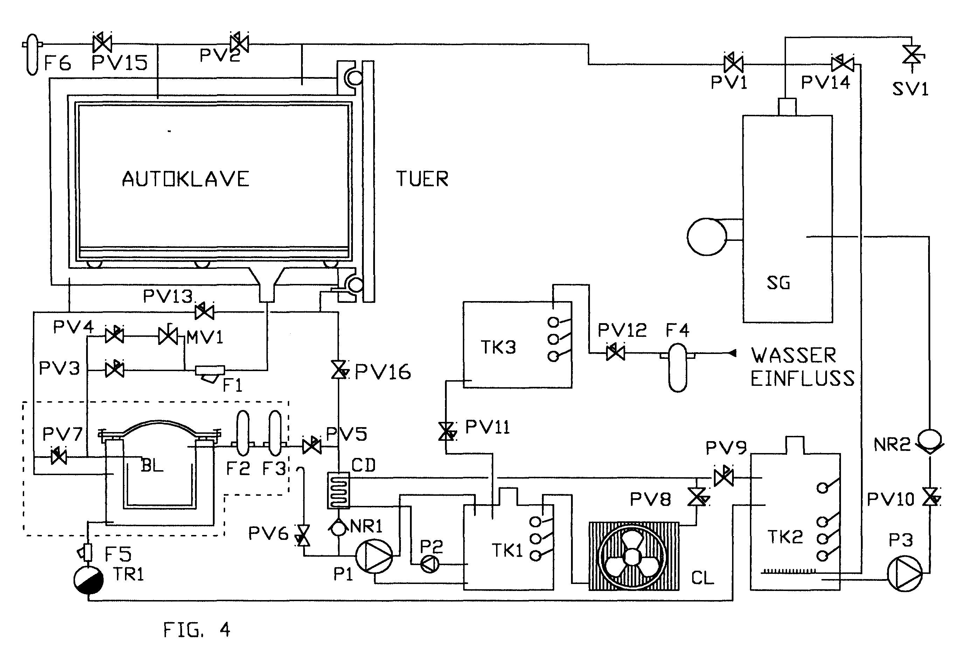

In Figur 4 sieht man die empfohlene Anordnung fluessiger Sterilisation mit dem Zusatz, der mit einer Trennlinie umrundet ist - d.h. des Verdampfers **BL**, des Vorfilters **F2**, des Sterilfilters **F3** - sowie die Anordnung der Wasserrueckgewinungsanlage, damit der Dampfgenerator beliefert wird.

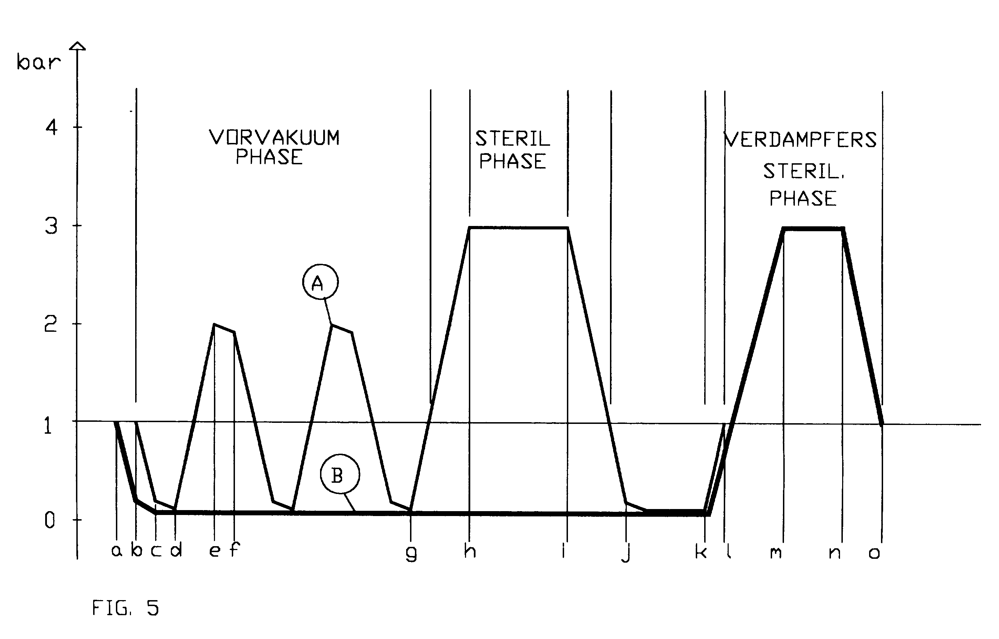

In Figur 5 sieht man ein Druckdiagramm der Kammer des Sterilisations-Autoklaven - Diagramm A - und der Kammer des Verdampfers - Diagramm B -, waehrend eines Sterilisations-Zyklus, wo sowohl der Verdampfer, als auch der Filter sterilisiert werden, damit die Sterilisations-Anordnung verstaendlicher wird.

Eine Funktion der Sterilisationsanordnung, die sich auf Figur 4 und 5 bezieht ist: Der Mantel des Autoklaven und der Mantel des Verdampfers BL sind miteinander verbunden. Am unteren Teil des Mantels des Verdampfers ist ein Wasserfilter und eine thermostatische Dampffalle angeschlossen, sodass die Kondensate und der kalte Dampf abfliessen koennen. In die Maentel wird Dampf eingelassen, dessen absoluter Druck durch ein Ventil **PV1** auf 3.5 bar beibehalten wird.

Wenn der Druck in den Maenteln normal ist, kann das Sterilisationsprogramm (Punkt **a**) beginnen. Die Pumpe **P2** beginnt, und das Ventil **PV8** oeffnet sich, sodass Wasser vom Kondensator **CD** durchfliessen kann, damit der ausfliessende Wasserdampf kondensiert wird. Die Vakuumpumpe **P1** beginnt ebenfalls, die Ventile **PV6** und **PV5** oeffnen sich, sodass im Verdampfer **BL** ein Vakuum entsteht. Alles o.e., was die Vakuumeinheit bildet und im weiteren so erwaehnt wird, bleiben in diesem Funktionszustand ab Punkt (**a**) bis Punkt (**k**) des Druckdiagrammes der Figur 5.

Wenn das Vakuum im Verdampfer den absoluten Druck von 0.2 bar erreicht hat, kann eine Auslaufkontrolle durchgefuehrt werden. D.h. die Vakuum-Einheit stoppt (die vorerwaehnten Ventile und Pumpen schliessen) fuer eine Minute, waehrend der Druck im Verdampfer staendig gemessen wird. Wenn sich der Druck ueber eine bestimmte Grenze hinaus aendert bleibt das Programm mit der Anzeige Undichtigkeit des Verdampfers - Filter, stehen.

Wenn die Dichtigkeitskontrolle des Verdampfers - Filter in Ordnung ist, beginnt die Vakuum-Einheit wieder (alles, die vorerwaehnten Ventile und die Pumpen) und der Sterilisations-Zyklus startet. Das Ventil **PV 13** oeffnet sich, damit sich die Tuer des Autoklaven schliesst und das Ventil **PV 3** oeffnet, damit in der Kammer des Autoklaven ein Vakuum entsteht (Punkt **b**). Wenn der Druck in der Kammer den absoluten Druck von 0.2 bar erreicht (Punkt **c**), geht die Vakuumbildung in der Kammer des Autoklaven fuer einige Zeit weiter bis zu Punkt **d**, damit die Luft von der zu sterilisierenden Ladung entnommen wird. Danach schliesst das Ventil **PV 3** und es wird ueber das Ventil **PV 2** Dampf in die Kammer des Autoklaven eingelassen, bis der absolute Druck der Kammer 2 bar erreicht, Punkt **e**, und die **PV 2** schliesst. Die Situation bleibt fuer einige Zeit so bestehen, sodass Dampf in die Ladung eingelassen wird. An Punkt **f** oeffnet sich wieder das Ventil **PV 3**, der Dampf wird in die Kammer des Verdampfers hineingelassen, eventuell vorhandehe Fluessigkeiten und die Kondensate werden vom Dampf mitgerissen, und der Prozess wird wie o.e. 3-4 mal wiederholt.

Dieser Puls des Drucks - Vakuums heisst Vorvakuum-Phase. Alles, was aus der Kammer des Autoklaven herausfliesst (Fluessigkeit, Luft, Dampf), geht in den Verdampfer **BL**, der sich die ganze Zeit ueber in einem Vakuum-Zustand befindet. Auf der einen Seite werden die Fluessigkeiten an die Grundflaeche des Verdampfers gefuehrt und verdampft, auf der anderen Seite werden alle Gase durch die Filter **F2**, **F3** filtriert und werden nun durch die Vakuum-Einheit (Kondensator - Vakuumpumpe) sterilisiert herausgelassen.

Am Ende der Phase des Vor-Vakuums, bei Punkt **g**, wird in die Kammer des Autoklaven Dampf durch das Ventil **PV 2** eingelassen, bis die erwuenschte Sterilisations- oder Desinfektionstemperatur erreicht wird. In unserem Beispiel 134 Grad C, d.h. ungefaehr 3.1 bar absoluter Druck. An Punkt **h** oeffnet sich das Ventil **PV 4**, das wieder mit der regelbaren Schalter **MV 1** verbunden ist, sodass eine kleine Menge Dampf oder Fluessigkeit von der Grundflaeche des Autoklaven zum Verdampfer ueberfliesst, und zwar waehrend der ganzen Dauer der Sterilisationsphase, sodass der Dampf in der Kammer des Autoklaven immer erneuert wird. Waehrend der Sterilisationsphase, von Punkt **h** bis **i**, wird die Temperatur der Kammer bestaendig beibehalten, indem das Ventil **PV 2** kontrolliert wird.

Zum Schluss der Zeitdauer der Sterilisation, Punkt i, schliessen die **PV 2**, **PV 4** und die **PV 3** oeffnet, sodass der Dampf in der Kammer zum Verdampfer hinausstroemt und es entsteht ein neues Vakuum im Raum des Autoklaven, das Trockenvakuum, womit bezweckt wird, dass die nun sterilisierte Ladung trocknet. Dieses Vakuum bleibt fuer einige Zeit beibehalten, von Punkt **j** bis **k**.

An Punkt **k** schliesst das Ventil **PV 3**, die Vakuumpumpe stoppt, die Ventile **PV 5**, **PV6** schliessen und 2 Prozesse beginnen:
a) das Ventil **PV 15** oeffnet, sodass in die Sterilisationskammer sterilisierte Luft durch das spezielle Filter **F 6** eindringt, bis der absolute Druck in der Kammer 1 bar erreicht (atmosphaerischer Druck), waehrend gleichzeitig die **PV 13** schliesst und die **PV 14** oeffnet, damit sich die Tuer des Autoklaven oeffnen kann. An Punkt **l**, wo der Druck 1 bar ist und die Tuer sich geoeffnet hat, kann der Autoklav entladen werden.
b) das Ventil **PV 7** oeffnet und es dringt Dampf in den Verdampfer und in die Filter ein, bis der absolute Druck im Verdampfer 3.1 bar erreicht, Punkt **m**, dieser Druck wird fuer 5 Minuten bestaendig beibehalten. Diese Phase wird als Sterilisationsphase des Verdampfers bezeichnet. An Punkt **n** schliesst die **PV 7**, die Vakuumpumpe beginnt, die Ventile **PV 5**, **PV 6** oeffnen und funktionieren bis zum Punkt **o**.

Mit dem Prozess, der bis jetzt dargestellt wurde, wird die Ladung des Autoklaven sterilisiert, waehrend alle Ausgangsstoffe des Autoklaven zuerst in den Verdampfer kommen. Die Fluessigkeit wird im Verdampfer schnell verdampft, aufgrund der Temperatur und des Vakuums, die dort herrschen. Der Dampf und die Luft werden in den Filtern **F2** und **F3** filtriert und kommen nunmehr sterilisiert heraus und in den Kondensator, wo der Wasserdampf kondensiert (weil er abgekuehlt wird) und verfluessigt wird. Zuletzt kommen die Luft und das Wasser in die Vakuumpumpe, wo sie in die Umwelt befoerdert werden koennen, ohne dass Gefahr fuer eine sekundaere Infektion besteht. Waehrend des Verdampfens im Verdampfer bleiben feste Stoffe im Behaelter zurueck, der sich an der Grundflaeche des Verdampfers befindet. Dieser Behaelter kann regelmaessig gefahrlos gereinigt werden, da diese Ueberreste sterilisiert sind.

Hier wird ein Sterilisationsprozess beschrieben, damit die Funktion der Erfindung im Zusammenhang mit dem Sterilisations-Autoklaven verstaendlich wird. Dies schliesst die Funktion der Erfindung mit einem anderen Sterilisations- oder Desinfektions-prozess mit gesaettigtem Dampf nicht aus. Nun wird eine Entsorgungsart des bereits sterilisierten Wassers beschrieben (ohne dass eine andere Art ausgeschlossen wird), fuer die bessere Funktion des Dampfgenerators mit Hilfe von Figur 4. Die **P 1** ist eine Fluessigkeitsring- Vakuumpumpe, sodass fuer ihre Funktion Wasser benoetigt wird. Dieses Wasser kommt vom Wasserbehaelter **TK 1**. Der Abfluss der Vakuumpumpe wird wieder zum Behaelter **TK 1** befoerdert, sodass auch das bereits sterilisierte Wasser, das aus dem Kondensator fliesst, in den Behaelter **TK 1** gefuehrt wird. Im gleichen Behaelter gibt es einen anderen Wasserzyklus, der durch die Wasserpumpe **P 2** den Kondensator mit Wasser versorgt, damit die Kondensierungen gekuehlt werden, und durch das Ventil **PV 8** wird das Wasser in den Kuehler **CL** gefuehrt, wo das Wasser luft- oder auf einer anderen Weise gekuehlt wird und zuletzt wieder in den Behaelter **TK 1** befoerdert wird. Damit die Vakuumpumpe richtig funktioniert braucht der Kondensator auch kaltes Wasser. Aus diesem Grunde funktioniert der Kreislauf Kondensator - Kuehler andauernd, damit das Wasser des Behaelters **TK 1** gekuehlt wird. Wenn der Behaelter **TK 1** mit Wasser aufgefuellt werden muss, oeffnet sich das Ventil **PV 11** und wird mit Wasser vom Wasserspeicher **TK 3** oder von der Wasserleitung aufgefuellt. Die Auffuellung des Dampfgenerators mit Wasser findet durch die Wasserpumpe **P 3** und des Ventils **PV 10** statt, vom Hilfswasserbehaelter **TK 2**, der mit Dampf vom Dampfgenerator durch das Ventil **PV 14** vorgewaermt wird, sodass die Dampfproduktion nicht jedes Mal, wenn der Dampfgenerator mit Wasser aufgefuellt werden muss, verlangsamt wird. Jedes Mal, wenn der Behaelter **TK 2** mit Wasser aufgefuellt werden muss, schliesst das Ventil **PV 8** und das **PV 9** oeffnet sich, so wird das Wasser vom Behaelter **TK 1** in den **TK 2** gefuehrt.

Auf dieser Weise haben wir die Rueckgewinnung des Wassers, das voellig ungefaehrlich ist, aufgrund dass es sterilisiert wurde, erreicht, damit der Dampfgenerator beliefert wird. Dies ist besonders fuer mobile Sterilisationseinheiten nuetzlich, aber auch in Ortschaften, die Probleme mit der Wasserversorgung oder mit zu hartem Wasser haben, wo dann die Enthaertung des Wassers noetig ist, um den hydraulischen Zyklus zu schuetzen.

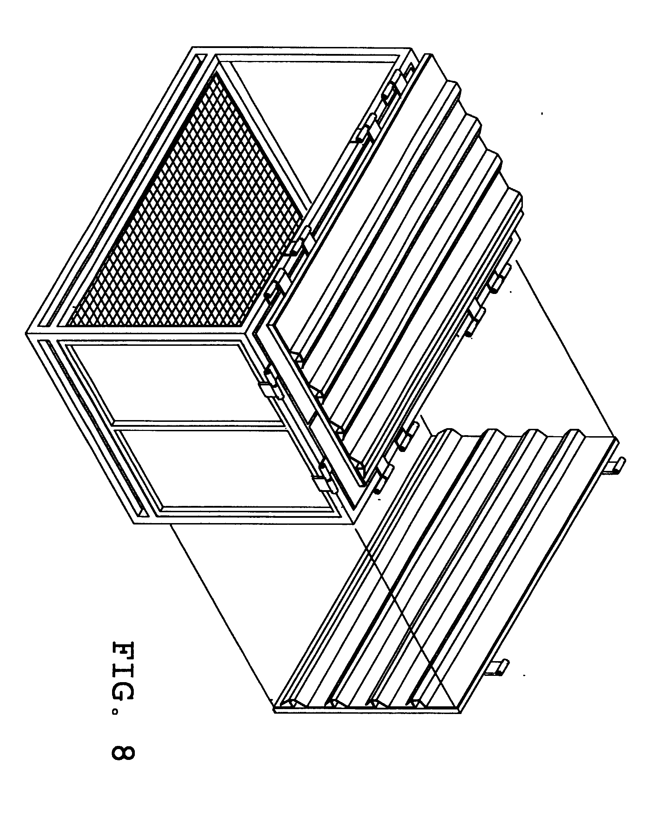

Mit Hilfe der Figuren 8 (dreidimensionale Ansicht), 7A - 7B (Schnittstellen), 6A-6B-6C (Seiten) wird ein Ladebehaelter fuer infektioese Abfaelle beschrieben. Damit die Moeglichkeit besteht, den Autoklaven mit infektioesen Abfaellen zu beladen, kann ein fahrbarer Behaelter benutzt werden, dessen Masse fuer die Kammer des Autoklaven geeignet sind. Die Grundflaeche des Behaelters ist dicht und auf der einen Seite, wo sich der Abfluss der Fluessigkeit des Behaelters befindet, geneigt. Dieser Abfluss schliesst sich mechanisch, ueber ein System mit einem mechanischen Ventil, mit einer Feder, die ueber eine geeignete elastische Dichtung verfuegt, um den Abfluss abzudichten und um die Temperatur auszuhalten. Der Abfluss des Behaelters befindet sich an einem solchen Platz, sodass er sich, wenn der Behaelter in die Kammer eingebracht wird, direkt ueber dem Abfluss der Sterilisationskammer befindet. Wenn sich der Behaelter an diesem Platz befindet, oeffnet sich mit Hilfe eines geeigneten Mechanismus, der sich an der Grundflaeche der Sterilisationskammer befindet, mechanisch das Ventil des Behaelters (es wird geschoben) und die Fluessigkeit, die sich in ihm befindet, wird in den Abfluss des Autoklaven gefuehrt.

An der Grundflaeche des Behaelters befindet sich eine durchloecherte waagerechte Flaeche von geeigneter Struktur und Staerke, sodass sie die Ladung der infektioesen Abfaelle aushalten kann. Auf dieser Flaeche befindet sich ein sehr duenner Sieb, sodass an der unteren Seite der Grundflaeche (an der geneigten Flaeche) nur fluessige und keine festen Stoffe (Abfaelle) durchlaufen koennen. Auf dieser Weise wird das Ventil des Behaelters geschuetzt, wie auch das ganze System des Autoklaven.

Der Behaelter ist so entworfen, dass er vor der Sterilisation beladen und danach wieder leicht entladen wird. Eine oder mehrere Seiten des Behaelters sind so eingerichtet, dass Dampf in den Behaelter eindringen kann, dass jedoch keine Fluessigkeit oder feste-Stoffe vom Behaelter austreten koennen.

Dies ist notwendig, weil es in den infektioesen Abfaellen Flaschen mit Fluessigkeiten oder geschlossene Saecke oder Packungen, die aufgehen, gibt, sodass fluessige oder andere Abfaelle gegen die Seitenwaende des Behaelters geschleudert werden.

Alle Seitenwaende koennen abnehmbar sein, damit der Behaelter gereinigt wird.

## Patentansprüche

1. Anordnung fuer den Schutz der Umwelt vor sekundaerer Infektion, die von den Ausgangsstoffen fluessiger Sterilisations-Autoklaven (mit Dampf) kommt, so definierbar, dass sie einen Verdampfer, ein Vorfilter und ein Sterilfilter fuer Gase beinhaeltet, die zwischen dem Abfluss der Sterilisationskammer des Autoklaven und der Vakuum-Einheit (Kondensator - Vakuumpumpe) eingeschaltet werden, sodass die Ausgangsstoffe der Autoklaven fluessiger Sterilisation verdampfen, dann in den speziellen Filtern (Vor- und Sterilfilter) durch Filtrieren sterilisiert werden, dann in die Vakuum-Einheit kommen, und zuletzt in die Umwelt (Atmosphaere, Abwasserkanaele) gelangen.

2. Anordnung fuer den Schutz der Umwelt nach Anspruch 1, so definierbar, dass der Verdampfer irgendeine Form haben kann, geeignet fuer die Arbeitsbedingugen und unabhaengig von der Heizungsart ist, die die Ausgangsstoffe der Autoklaven fluessiger Sterilisation verdampfen kann, und zusaetzlich ueber einen geeigneten abnehmbaren Behaelter verfuegt, sodas die festen Ueberreste nach dem Verdampfen zurueckbleiben und gereinigt werden.

3. Anordnung fuer den Schutz der Umwelt nach Anspruch 1, so definierbar, dass das Vorfilter und das Sterilfilter irgendeines Typs sein kann, der Vorfilter dafuer geeignet ist, dass er feste Koerper zurueckbehaelt, Sterilfilter, dafuer geeignet ist, dass er Gase sterilisiert und fuer die Funktionstemperatur geeignet ist.

4. Sterilisations- oder Desinfektionsprozess mit einem Autoklaven fluessiger Sterilisation, so definierbar, dass im Prozess die Einzelheiten von Anspruch 1 miteinbezogen werden, d.h. der Verdampfer, das Profilter und das Sterilfilter, sowie den Sterilisationsprozess dieser Teile.

5. Anordnung fuer die Wasserrueckgewinnung im Autoklaven fluessiger Sterilisation, die ueber das System des Umweltschutzes aus Anspruch 1 verfuegen, so definierbar, dass die fluessigen Ausgangsstoffe dieser Autoklaven wieder dem Dampfgenerator zugefuehrt werden koennen.

6. Art und Weise des Umweltschutzes durch die Entsorgung infektioeser Abfaelle, so definierbar, dass die infektioesen Abfaelle in Autoklaven fluessiger Sterilisation sterilisiert oder desinfiziert werden koennen, die die Umwelt nicht infizieren, d.h. die ueber die Einzelheiten aus Anspruch 1 und 4 verfuegen sodass die Abfaelle danach ueber den gewoehnlichen Weg der Hausmuellentsorgung beseitigt werden koennen. Die Sterilisation oder Desinfektion der infektioesen Abfaelle koennte ueber eine stationaere oder mobile Einheit der Autoklaven fluessiger Sterilisation durchgefuehrt werden, gemaess Anspruch 1 oder gemaess Anspruechen 1, 4 und 5.

7. Ladebehaelter fuer Autoklaven fluessiger Sterilisation mit infektioesen Abfaellen, gemaess Anspruch 6, so definierbar, dass er so eingerichtet ist, dass Dampf von den Seitenwaenden eindringen kann, dass keine Fluessigkeit oder festen Stoffe von seinen Seitenwaenden herauskommen koennen, dass er leicht gereinigt werden kann, dass nur die Fluessigkeiten von seiner Grundflaeche an einem geeigneten Punkt hinausfliessen koennen, sodass diese direkt in den Abfluss der Kammer des Autoklaven fliessen, und die Fluessigkeiten nur dann hinausfliessen koennen, wenn sich der Behaelter an seinem Platz in der Sterilisationskammer befindet.
